# EUROPEAN PATENT SPECIFICATION

(11) **EP 3 400 438 B1**
(45) Date of publication and mention of the grant of the patent: **28.07.2021**
(21) Application number: 16715617.3
(22) Date of filing: 09.03.2016
(51) Int. Cl.: G01N 33/497

(54) **METHOD FOR DETECTION OF MOULD CONTAMINATION IN GRAIN**
VERFAHREN ZUM NACHWEIS VON SCHIMMELKONTAMINATION IN GETREIDE
PROCÉDÉ DE DÉTECTION DE CONTAMINATION DE CÉRÉALES PAR DES MOISISSURES

(30) Priority: 06.01.2016 LT 2016001
(43) Date of publication of application: 14.11.2018
(73) Proprietor: Gamtos Tyrimu Centras, LT-08412 Vilnius (LT)
(72) Inventor: BUDA, Vincas, LT-14159 Vilniaus raj. (LT); BUTKIENE, Rita, LT-02184 Vilnius (LT); BLAZYTE-CERESKIENE, Laima, LT-14284 Vilnius (LT); PECIULYTE, Dale, LT-07118 Vilnius (LT); APSEGAITE, Violeta, LT-07124 Vilnius (LT)
(74) Representative: Gerasimovic, Liudmila
(86) International application number: PCT/IB2016/051320
(87) International publication number: WO 2017/118881

(56) References cited:
- WO-A2-2012/021531
- US-A1- 2006 280 765
- JASSY DRAKULIC ET AL: "Sharing a Host Plant (Wheat [Triticum aestivum]) Increases the Fitness of Fusarium graminearum and the Severity of Fusarium Head Blight but Reduces the Fitness of Grain Aphids (Sitobion avenae)", APPLIED AND ENVIRONMENTAL MICROBIOLOGY, vol. 81, no. 10, 15 May 2015 (2015-05-15), pages 3492-3501, XP055284862, US ISSN: 0099-2240, DOI: 10.1128/AEM.00226-15
- MAGAN N.; EVANS P.: "Volatiles as an indicator of fungal activity and differentiation between species, and the potential use of Electronics nose technology for early detection of grain spoilage", J. STORED PROD, vol. 36, 2000, pages 319-340, XP002759386, cited in the application

## Description

### Field of the invention

Cereals can be often contaminated with spoilage or pathogenic microorganisms making many products no more edible or affecting their taste by the production of undesirable flavours. These microorganisms can produce toxic substances causing a serious hazard for human and animal health besides a very high economic loss.

The present invention relates to a rapid and sensitive method for determining mould contamination in grain. Particularly, this invention relates to detection of presence or absence of metabolites of mould fungi in grain by gas chromatography and insect electroantennography performed simultaneously and evaluating insect electroantennographic response at retention time corresponding to that of standard known to be produced by grain contaminating mould fungi.

### Background of the invention

Currently, many techniques for detection of mould contamination are used including molecular methods, latex agglutination tests, enzyme linked immunosorbent assay (ELISA), bioluminescence (patents CN 102094080, RU 2079128 among others). Other currently available methods include: microscopic examination, culture on agar, detection of fungal metabolites, such as chitin, ergosterol or mycotoxins using chromatography (WO 2013/135889; US patent application No.2014/0356978, etc.) or Raman spectroscopy (Grow A.E., Wood L.L., Claycomb J.L. et al. 2003. New biochip technology for label-free detection of pathogens and their toxins. J. Microbiol. Methods. 53: 221-233).
US patent No. 8,127,593 discloses method of detecting of fungal contaminants of indoor surroundings such as buildings, premises etc. by the presence of certain predetermined volatile organic compounds (VOCs).
European patent No EP2568283 discloses method for determining mycotoxins in any environment based on chemical "fingerprints" analysis and includes at least a set of VOCs molecules associated with mycotoxicogenese.

However, all these methods are not always applicable for both the cost and labour time required to analyse a sample, as well as for necessity of skilled operators. Besides, some strains of grain contaminating moulds do not produce toxins, thus direct analysis of noxious metabolites leads to false conclusion on presence/absence of fungal contamination. Electronic nose has been applied to detect fungal volatiles (Magan N., Evans P. 2000. Volatiles as an indicator of fungal activity and differentiation between species, and the potential use of Electronics nose technology for early detection of grain spoilage. J. Stored Prod. 36: 319-340) however, despite high sensitivity of the method, it is non-specific and too much dependent on environment.

With the aim to detect namely grain contamination and monitor quality loss, it was suggested to analyse appearance of volatiles as an indicator (Stawicki S., Kaminski E., Niewiarowicz A. et al. 1973. The effect of microflora on the formation of odours in grain during storage. Annales de Technologie Agricole 22: 449-476; Kaminski E., Stawicki S., Wasowicz E. 1974. Volatile flavour compounds produced by moulds of Aspergillus, Penicillium and fungi imperfecti. Appl. Microbiol. 27: 1001-1004.; Abramson D., Sinha, R.N., Mills, J.T. 1980. Mycotoxin and odor formation in moist cereal grain during granary storage. Cereal Chemistry 57: 346-351).
After studying many grain samples, it has been determined that certain common patterns of volatile emissions appear when analyzing known mouldy samples. In particular, if the volatiles are extracted from such samples and analyzed, there is a large abundance of alcohols, aldehydes and ketones (Magan N., Evans P. 2000. Volatiles as an indicator of fungal activity and differentiation between species, and the potential use of Electronics nose technology for early detection of grain spoilage. J. Stored Prod. 36: 319-340). The method applied was not allowing detection of mould in grain using only single compound as specific biomarker.

As indicators of grain contamination by fungi, a group C₇, C₈ and C₉ VOCs were suggested to use (US 4,314,027) as well as many other groups of volatiles (WO 2013/135889). For detection and analysis of the compounds either GC or GC-MS (gas chromatography-mass spectrometry) technique was used. For application of the methods high expenditure of chemicals including those of extra high purity (standards) is needed.

On the other hand electroantennography (EAG) is known as a technique for the detection of volatiles perceived by the antennal olfactory apparatus of insects. The method is based on voltage fluctuations between the tip and base of an insect antenna during stimulation with VOCs, e.g. kairomones. For example, EP0852723 discloses biosensor system which includes insect antenna for measuring one or more trace organic components in air resulting from crop damage by pest insect.

Gas chromatograph (GC) coupled with an electroantennographic detector (EAD), also known as GC-EAD to be mentioned as a technique for elucidation of insect semiochemicals (Arn H., Stadler E., Rausfcher S. 1975. The electroantennographic detector: a selective and sensitive tool in the gas chromatographic analysis of insect pheromones. Z. Naturforsch. 30C: 722-725; Baker T.C., Francke W., Millar J.G. et al. 1991. Identification and bioassay of sex pheromone components of carob moth, Ectomyelois ceratoniae (Zeller). J. Chem. Ecol. 17: 1973-1988).
Determination of plant pathogens by GC-MS and GC-coupled EAD was described for treating of wheat ears with aphids and *Fusarium graminearum* inoculum to monitor disease progress [Jussy Drakulic et al.:" Sharing a Host plant (Wheat [Triticum aestivum]) Incerases the fitness of Fusarium graminearum and the Severity of Fusarium Head Bligght but Reduces the Fitness of Grain Aphids (Sitobion avenae)", Applied and Environmental Microbiology, vol. 81, No. 10, publ. May 2015, pages 3492-3501]. Detection of mould contamination in grain under storage, using specific single biomarker is not disclosed.
The GC-EAD system comprises of an antenna placed between two electrodes from which an amplified signal is recorded by computer software. GC column effluent is split between the flame ionization detector (FID) and the EAD using splitter, thus allowing simultaneous recording by two detectors (one of those non-selective and another highly selective).
Use of analogous GC-EAD systems is disclosed in WO 2012/021531, publ. 16/02/2012 and US 2006/280765, publ. 14/12/2006 for various purposes.

According to the method of detecting mould toxin infestation of grain, disclosed in US patent No. 4,314,027, grain volatile compounds were absorbed on solid sorbent placed in special device. The only embodiment described is testing of corn sample known to be aflatoxin infected, where grain sample of 100 g used, which was vacuum stripped for 120 min. to collect some of VOCs. Heating up to 100 °C was necessary to draw away volatiles. To reveal group of C₇ - C₉ alcohols, ketones and aldehydes, i.e. volatiles indicative of the presence of mycotoxins, GC was used. A readout is provided indicating whether the offending C₇, C₈ and C₉ volatiles are present and they can be compared with a standard chart showing the readings if the offending volatiles are present.

To run the analysis many standards (C₇ to C₉ alcohols, ketones and aldehydes) are needed. As mentioned above, some grain contaminating moulds might produce metabolite(s) other than mycotoxins and absence of mould toxins by method disclosed might lead to wrong conclusion on absence of fungal contamination. In the analysis uncertainty remains if some or few (part) of the set compounds are lacking, thus conclusion on contamination becomes not strict.
Besides, as the correct conclusions depends on the total amount of VOCs collected (extracted from the sample), therefore according to the example presented, the total duration of VOC collection is not less than 2 h. Special nonstandard device for VOC collection is needed which should be made and adjusted to GC injection port as well as filled with special sorbent. Using just a single detector (no extra one e.g. MS or EAD) and comparing chromatograms of a sample and standard chart misleading conclusion can be drawn due to similar or even the same retention time of different compounds, e.g. terpenes and other organic compounds.

### Problem to be solved

It can be seen, that there is current need in fast and highly sensitive, however easy to interpret, method to detect a fungal contamination in grain, which would allow early and reliable detection of contamination, avoiding potential loss of grain production or part thereof.

### Summary of the invention

The principal object of the present invention is method for detection of mould contamination in grain, which comprises collecting of gaseous sample from the grain and treatment of sample collected by gas chromatography for detecting target volatile(s) in the sample. Method of present invention is characterised in (i) coupling of gas chromatography step with insect electroantennography step and (ii) determining the presence of mould contamination in grain by evaluating the antennal response at the retention time, corresponding to that of a single specific biomarker.
Said single specific biomarker, indicative of mould contamination, is 3-methyl-1-butanol.

Gas chromatography and insect electroantennography in the method proposed are performed simultaneously.
Normally flame ionization detection is performed parallelly with insect electroantennography by splitting gas chromatography effluent between flame ionization detector and electroantennography detector in the ratio 1:1.

In the preferred embodiments of present invention collecting of gaseous sample from the grain is performed at ambient pressure and temperature up to 40+5°C, extracting volatiles from up to 25 g amount of grain for at least 30 min.

In particular embodiments of invention the suitable antennae for use in electroantennography step is selected from the group of insects, comprising *Plodia interpunctella, Tenebrio molitor* L., *Geotrupes stercorarius* L., *Leptinotarsa decemlineata* (Say), *Drosophila melanogaster* (Meigen), *Musca domestica* L., *Coreus marginatus* L., *Vespula germanica* (Fabricius), and *Apis mellifera* L. , preferably *Drosophila melanogaster, Plodia interpunctella* and *Apis mellifera.*
In the most preferred embodiment of present invention the suitable antenna for use in electroantennography step is that of *Drosophila melanogaster.*

The method of present invention is applicable at least in cases, then the mould is selected from a group of fungi, comprising *Alternaria alternata, Alternaria brassicola, Aureobasidium pullulans, Aspergillus flavus, Aspergillus niger, Aspergillus ochrachloron, Aspergillus parasiticus, Aspergillus oryzae, Fusarium graminearum, Fusarium culmorum, Fusarium poae, Fuasarium sporotrichioides, Penicillium aurantiogriseum, Penicillium chrysogenum, Penicillium citrinum, Penicillium cyclopium, Penicillium digitatum, Penicillium funiculosum, Penicillium roquefortii, Penicillium raistricki, Penicillium viridicatum, Penicillium expansum, Scopulariopsis brevicaulis, Rhizopus oryzae* and *Gusarium* spp.

Another object of this invention is to provide a detection system for detection of mould infected grain which is highly sensitive, even sensitive to as low as at sub-microgram level. System for detection of mould contamination in grain by the method according to present invention comprises a solid phase microextraction device (SPME) and gas chromatograph (GC) coupled with an electroantennographic detector (EAD) in parallel with flame ionization detector (FID), wherein (GC) is equipped with a splitter, allowing the simultaneous recoding of (FID) and (EAD) signals.
An electroantennographic detector (EAD) for detection of mould contamination in grain according to present invention comprises an insect antenna placed between two electrodes, from which the amplified signal might be recorded by computer means, and airflow means for delivery of volatiles from (GC) to insect antenna.

One more important object of present invention is use of 3-methyl-1-butanol as single biomarker for electroantennographical detection of mould contamination in grain.

### Brief description of the drawing:

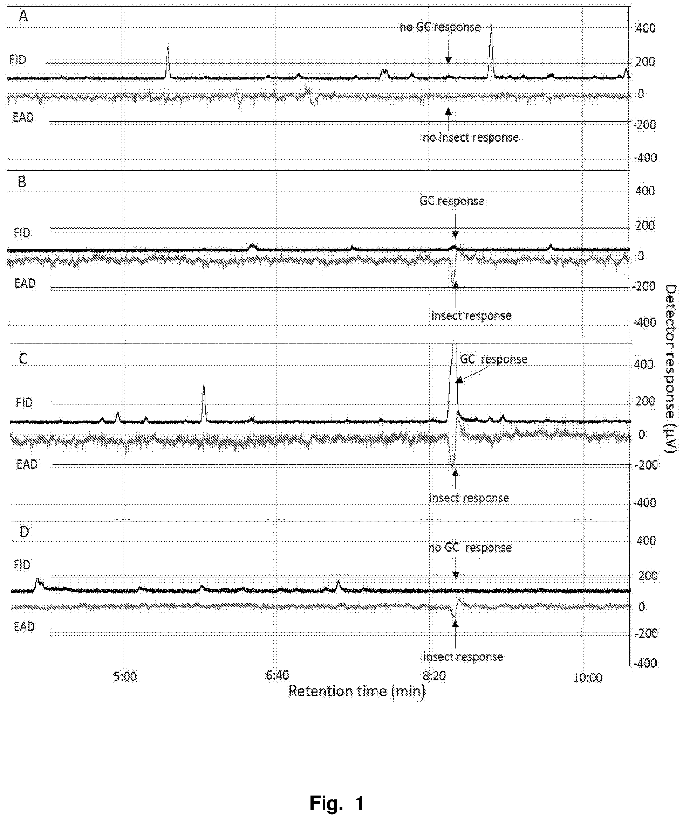

Fig. 1 represents simultaneous run of GC-EAD system detectors during analysis of volatiles collected from airspace of grain sample. Responses of GC (FID) and biological (EAD) detectors to the volatiles from:
A - uninfected grain (no response of both FID and EAD detectors in the absence of biomarker);
B - fungi infected grain (low response of FID and high response of EAD at low concentration of biomarker);
C - fungi infected grain (extremely high response of FID and high response of EAD at high concentration of biomarker);
D - fungi infected grain (no response of FID and response of EAD detector as concentration of biomarker is low but sufficient to detect by EAD however not sufficient for detection by FID).

### Detailed description of the Invention

As mentioned above the main object of present invention is a method for determination of mould contamination in grain using specific volatile compound produced by fungi. The method includes collection of air-born volatiles, gas chromatography (GC) and insect electroantennography (EAD) performed simultaneously. Air-born volatiles were injected into GC and simultaneously insect EAD was recorded. EAD reveals if air-born volatiles contain biomarker compound known to be produced by grain contaminating mould.
After study of numerous VOCs emitted by fungi contaminated grain we found that use of a single VOC is sufficient to detect contamination. The specific VOC or universal biomarker was revealed in the study of stored product insect pest's behaviour analysis, which revealed the key compound used by the insects to avoid fungi contaminated grain. Such key compound was surprisingly found to be 3-methyl-1-butanol; and EAD response to such biomarker was obtained in all cases studied where grain was infected with fungi. Moreover, response to 3-methyl-1-butanol appeared earlier, compare, e.g. to sesquiterpenes as claimed in EP2568283. Therefore 3-methyl-1-butanol might be treated as early indicator of mould contamination, comparing with other compounds, appearing at the deeper infection stage, when results of grain damage can already be seen visually. The standard 3-methyl-1-butanol was further used to reveal retention time of the compound in GC analysis.

Correspondently the system of detection of present invention comprises a solid phase microextraction (SPME) device, gas chromatograph (GC) coupled with an electroantennographic detector (EAD).
SPME device is portable and standard one, and it might be easily attached to GC-EAD system which suits for collection VOCs and injection to GC. The basic elements of GC-EAD set up are: gas chromatograph, insect antennal preparation, recording electrodes, amplifier, computer for recording/analysis and airflow means for VOC delivery from GC to insect antenna.

A sample of grain to be tested for the presence of mould is obtained in conventional manner. The sample is placed in glass container sealed with aluminium folia and kept under constant temperature to receive equilibrium of volatiles' concentration in headspace. Volatiles are extracted by absorption on solid phase coating using SPME device. The fibre retracts within the needle sheath for mechanical protection. The fibre is exposed and retracted selectively for sampling, and sample delivery, by a fibre holder. Using needle of fibre holder folia is penetrated and SPME fibre coated with divinylbenzene/polydimethylsiloxane (DVB/PDMS) or polydimethylsiloxane (PDMS) is exposed in grain headspace for a certain duration. The time of absorption is not critical and from 30 minutes to 60 minutes is generally sufficient. When the fibre is exposed in headspace, volatiles emitted from the grain sample are absorbed upon the fibre polymeric coating.
After absorption the fibre is retracted within the needle, SPME device is removed from grain sample and is inserted into injection port of a GC. During desorption carrier gas (hydrogen or helium) is used to carry volatiles absorbed into GC column. The column effluent is split between the flame ionization detector (FID) and the electroantennogram detector (EAD) using splitter. The EAD consists of an insect antenna (placed between two electrodes), amplifier and electronic device for control of airstream carrying volatiles to antenna. The electrodes are connected to the insect antenna via saline solution (e.g. KOH or Insect Ringer's solution) or electrogels, for example, those used for electrocardiograms. Saline solution and electrogel prevent dehydration and rapid healing of the injured insect tissues and enable recording of antennal electric signals. EAG recording is also possible either using whole insect or dissected head, in such case one electrode is connected to antennal tip and the other to the insect body. Volatile compounds from GC column following split are delivered both to FID and EAD (insect antenna). The split allows simultaneous recording and comparison of FID and EAD responses. Amplified EAD signal is recorded by computer software.

Volatiles are collected on SPME fibre coated with divinylbenzene/poly-dimethylsiloxane (DVB/PDMS) or polydimethylsiloxane (PDMS). With the aim to decrease absorption of less polar compounds (spam) PDMS coated fibre is preferable. Following establishing volatiles' emission equilibrium between grain and air, i.e. after 15 to 60 min period (minimal 15 min period is sufficient), collection of volatiles is started and lasts from 30 to 60 min. Further increase of collection period increases not only total duration of analyses, but due to competitive absorption-desorption processes it may negatively affect the analysis. Absorption can be carried out within temperature range from ambient to 40 ± 5 ºC. Lower temperature leads to insufficient emission of the compound claimed herein, per se, as biomarker, while higher temperature enhances desorption. Thus, temperature of 40 ºC is optimal for collection of the marker volatile, if present, for testing. For GC analysis may be used fused silica columns of various polarity such as 100 % polyethylene or 5 % phenyl methylpolysiloxane. Temperature program and flow rate necessary for suitable separation of the VOCs should be chosen. In injector port desorption duration may vary from 1 to 3 min., however, 1.5 min is the most suitable time for desorption of an analyte. Air flow speed above an insect antenna is within the range of 0.25 to 1.5 m/sec with the optimum at 0.5 m/sec.

The method is suitable to detect grain contamination by fungi, such as *Alternaria alternata, Alternaria brassicola, Aureobasidium pullulans, Aspergillus flavus, Aspergillus niger, Aspergillus ochrachloron, Aspergillus parasiticus, Aspergillus oryzae, Fusarium graminearum, Fusarium culmorum, Fusarium poae, Fuasarium sporotrichioides, Penicillium aurantiogriseum, Penicillium chrysogenum, Penicillium citrinum, Penicillium cyclopium, Penicillium digitatum, Penicillium funiculosum, Penicillium roquefortii, Penicillium raistricki, Penicillium viridicatum, Penicillium expansum, Scopulariopsis brevicaulis, Rhizopus oryzae, Gusarium* spp. and others.

In particular embodiments of invention the suitable were antennae of insects, at least selected from the group of *Plodia interpunctella, Tenebrio molitor* L., *Geotrupes stercorarius* L., *Leptinotarsa decemlineata* (Say), *Drosophila melanogaster* (Meigen), *Musca domestica* L., *Coreus marginatus* L., *Vespula germanica* (Fabricius), and *Apis mellifera* L.
However, *Drosophila melanogaster, Plodia interpunctella* and *Apis mellifera* are the most preferable insects as possess the highest sensitivity to biomarker used (3-methyl-1-butanol).

### Description of the embodiments of the invention

Represented below is information on specific examples, which are presented for illustrative purpose and are not limiting the scope of the present invention.

### Example 1. Volatile collection from grain samples

The grain sample is placed in glass container sealed with triple aluminium folia and kept under constant 40 °C temperature for 0.5 h and following volatile absorption using SPME procedure during 1 h. VOCs were absorbed by polydimethylsiloxane (PDMS, 100 µm, Supelco, USA) fibre. After that immediately desorption for 1.5 min in GC is performed and both GC and insect EAG are recorded (GC-EAD).

### Example 2. GC-EAD

VOCs absorbed on SPME fibre is used in GC-EAD analysis. The GC-EAD was performed using a Clarus 500 gas chromatograph (PerkinElmer, Waltham, MA, USA) equipped with a DB-Wax capillary column (30 m; 0.25 mm i.d., 0.25 µm film-thickness, Agilent Technologies, Santa Clara, CA, USA) and a 1:1 effluent splitter that allowed a simultaneous flame ionization detection (FID) and EAD of the separated VOCs.

Injection port temperature and transfer line temperature was 240 °C. The analyses were performed using the following temperature program: initial 40 °C held for the first 2 min, then rising to 200 °C at 5 °C/min, then rising to 240 °C at 10 °C/min and finally 240 °C held for 14 min. Hydrogen was used as carrier gas (2.5 mL/min).

The effluent from the column was split into two parts, one transferred to FID and the other to EAD in ratio 1:1. Antenna of the insect was used as a detector to detect electrophysiologically active volatile compounds. An insect antenna excised at its base was mounted on a glass capillary indifferent electrode filled with 0.9 % NaCl saline (Ilsanta, Lithuania) and grounded via a silver (Ag) wire. A recording electrode consisted of a glass capillary filled with the solution (Ag wire) was brought into contact with the distal end of the antenna. This preparation was connected to a high-impedance IDAC-232 amplifier (Syntech, The Netherlands) with automatic baseline drift compensation, which was interfaced to PC equipped with program GcEad V. 4.4 (Synthech, NL 1998). Compounds that elicited consistent responses were considered as EAD active.

Antennally active compound 3-methyl-1-butanol was identified by comparison of the retention time with that of the available authentic synthetic compound.

### Example 3. The most suitable insect-detectors

To find out the most sensitive insect-detector for 3-methyl-1-butanol as fungal biomarker the electrophysiological responses of nine insect species antennae were tested. Antennae were prepared for testing the same way as indicated in Example 2.

The test compound dissolved in hexane (10 µL) was deposited on a filter paper strip (5 x 45 mm) (Whatman®1, England). After solvent evaporation at room temperature, the filter paper was inserted into Pasteur pipette. For stimulation, an airflow (7 mL/s) carrying the odour stimulus was inserted for 0.5 s into a continuous airflow (20 mL/s) by using a stimulus controller (CS 55; Syntech, The Netherlands). Six concentrations of 3-methyl-1-butanol were tested in the ascending order (0.1, 1, 10, 10², 10³, then 10⁴ µg). A solvent blank (10 µL of hexane after evaporation) was tested as a control stimulus before application of the lowest as well as after the highest dosage of the test compound. Peak voltage amplitude was recorded during the puff delivery of each stimulus as an antennal response (mV). Each stimulation was followed by a minimum of a 1 min purge period of filtered air to ensure recovery of antennal olfactory receptors.

Grain pest *P. interpunctella,* honey bee *A. mellifera,* and fruit fly *D. melanogaster* were the most sensitive insects to 3-methyl-1-butanol. Surprising effect of top sensitivity demonstrated *D. melanogaster,* fruit flies absolutely not related to stored grain. Beetles *T. molitor, G. stercorarius,* and *L. decemlineata,* as well as fly *M. domestica,* bug C. *marginatus,* and wasp *V. germanica* were approximately 100 times less sensitive to the compound.

### Example 4. Detection of the presence or the absence of fungal VOCs

In the following example, 25 g samples of wheat were tested. A first sample was a sample of wheat known to be non-contaminated. Other samples used were the samples contaminated in the laboratory by wheat inoculation either with *Aspergillus flavus* or *Alternaria alternata* mould.
VOCs of each sample were collected using SPME as described in Example 1. Absorbed VOCs were used in GC-EAD analysis, which is described in Example 2, to determine the presence or absence of 3-methyl-1-butanol, indicative of the mould contamination. The results can be seen on Fig. 1. Non-contaminated sample show no peak in both GC and EAD traces at retention time characteristic for of 3-methyl-1-butanol, where no response of both FID and EAD detectors can be seen as 3-methyl-1-butanol is absent; and it means "no mould contamination" (Fig. 1, A). Mould contaminated with *Aspergillus flavus* (Fig 1, B), *Alternaria alternata* (Fig. 1, C) samples show peak in both GC and EAD traces at retention time chatracteristic for 3-methyl-1-butanol, and it means "mould contamination is present" as concentration of 3-methyl-1-butanol is low (Fig. 1, B) or concentration of 3-methyl-1-butanol is extreme (Fig. 1, C).
The grain sample contaminated by *Aspergillus flavus* (Fig. 1, D) contained level of 3-methyl-1-butanol so low that it was below GC detection level, however, it was detected by EAD, thus, it means "mould contamination is present". The sample demonstrate higher sensitivity of EAD.
Analogous results were obtained, when testing grain contaminated either by *Fusarium graminearum, Penicillium aurantiogriseum* or other fungi.

High effectivity of the method is achieved due to:
Extremely high sensitivity, sufficient VOC of ppb order in the headspace;
Simple analysis, which is based on the single marker compound only, and no index calculations are needed compare to known methods based on many compounds' analyses; Absence of uncertainties (present in many cases based on indices' calculations); Standard equipment is needed only;
For EAD recording the most suitable insect *Drosophila melanogaster* used is easy to rear and maintain in culture.

From the above it can be seen that the invention proposed exhibits some new advantages, the main being:
1. Detection of fungal contamination is rapid and simplified since analysis of limited number of volatiles (just a single compound) is sufficient.
2. The resulting data are not overcrowded by information; high skill of operator is not necessary to read and understand the records.
3. Simultaneous increase of sensitivity lead to early detection of contamination, thus allow to avoid potential loss of grain production.

New technical approach allows to collect volatiles from grain sample by common solid phase microextraction technique (SPME) at normal pressure and temperature of about ambient. Small amount of grain (up to 25 g) is sufficient. In the process of grain contamination analysis, two detectors are used, namely flame ionization (FID) and electroantennogram (EAD), thus reliability and accuracy of mould detection increases.
Minimal number of high purity standards is sufficient, just a single.
Sensitivity of the suggested method was increased comparing to known ones (as US patent No. 4,314,027) due to supplement of insect EAD. Amount of ppm order of a compound is needed to record FID response, and for insect EAD less than range of one to few ppb of a compound is sufficient, i.e. appr. 1000 time less. MS analysis and corresponding equipment are not involved. No need to apply vacuum and heat a sample above 40 °C. Shorter duration of GC run (approximately 10 min) and simple visual analysis of records.

In case when both FID and EAD responses are present, fungus contamination is at ppm level, while in case when FID response is absent (noise level) and EAD response is present, fungus contamination is much lower, and reaches ppb level.
Method can be run with FID off as well.

The invention is advantageous comparing to known methods for grain contamination detection (mentioned above).
- The duration from sample collection to the end of analysis takes approximately 45 min (up to 1 hour), i.e. it is shorter compare to that needed for methods mentioned above.
- The method gains advantage in possibility to detect contamination by such fungi strains which do not produce toxins (for example, *Aspergillus ochraceus* strains such as KA-95, KA-101, KA-284).
- Analysis is more simple comparing to those of known methods as it is enough to detect (and analyse) presence of a single marker only.
- Sensitivity is higher compare to that available using technique suggested (known), as the latter do not include biological (the most sensitive) part of detection.
- Analysis is available not under laboratory conditions, but also at a grain store or elevator when standard portative equipment might be used (GC-EAD).
The method is environmentally friendly; no organic solvents are needed.

### Industrial applicability

The invention can be useful in agriculture for evaluation of grain storage within elevators; for evaluation of animal forage quality; in food industry for evaluation of cereal quality in flour and breadstufs production, etc.

## Claims

1. Method for detection of mould contamination in stored grain, which comprises collecting of gaseous sample from the grain and treatment of sample collected by gas chromatography for detecting target volatile(s) in the sample, ***characterised in*** (i) coupling of gas chromatography step with insect electroantennography step and (ii) determining the presence of mould contamination in grain by evaluating the antennal response at the retention time, corresponding to that of a single specific biomarker, wherein said single specific biomarker, indicative of mould contamination, is 3-methyl-1-butanol.

2. The method according to claim 1, ***characterised in that*** the suitable antenna for use in electroantennography step is selected from the group of insects, comprising *Plodia interpunctella, Tenebrio molitor* L., *Geotrupes stercorarius* L., *Leptinotarsa decemlineata* (Say), *Drosophila melanogaster* (Meigen), *Musca domestica* L., *Coreus marginatus* L., *Vespula germanica* (Fabricius), and *Apis mellifera* L. , preferably *Drosophila melanogaster, Plodia interpunctella* and *Apis mellifera.*

3. The method according to claim 2, ***characterised in that*** the suitable antenna for use in electroantennography step is that of *Drosophila melanogaster.*

4. The method according to any of preceding claims, ***characterised in that** gas* chromatography and insect electroantennography are performed simultaneously.

5. The method according to claim 4, ***characterised in that*** flame ionization detection is performed parallelly with insect electroantennography by splitting gas chromatography effluent between flame ionization detector and electroantennography detector in the ratio 1:1.

6. The method according to any one of the preceding claims, ***characterised in that*** collecting of gaseous sample from the grain is performed at ambient pressure and temperature up to 40±5°C, extracting volatiles from up to 25 g amount of grain for at least 30 min.

7. The method according to any one of the preceding claims, ***characterised in that*** mould is selected from a group of fungi, comprising *Alternaria alternata, Alternaria brassicola, Aureobasidium pullulans, Aspergillus flavus, Aspergillus niger, Aspergillus ochrachloron, Aspergillus parasiticus, Aspergillus oryzae, Fusarium graminearum, Fusarium culmorum, Fusarium poae, Fuasarium sporotrichioides, Penicillium aurantiogriseum, Penicillium chrysogenum, Penicillium citrinum, Penicillium cyclopium, Penicillium digitatum, Penicillium funiculosum, Penicillium roquefortii, Penicillium raistricki, Penicillium viridicatum, Penicillium expansum, Scopulariopsis brevicaulis, Rhizopus oryzae* and *Gusarium* spp.

8. Use of 3-methyl-1-butanol as single specific biomarker for electroantennographical detection of mould contamination in stored grain, wherein electroantennographical detection is coupled with gas chromatography.

## Patentansprüche

1. Verfahren zum Nachweis von Schimmelkontamination in gelagertem Getreide, bei dem man eine gasförmige Probe von dem Getreide entnimmt und die entnommene Probe zum Nachweis von flüchtigen Zielkomponenten in der Probe einer Gaschromatografie unterzieht, **dadurch gekennzeichnet, dass** (i) der Gaschromatografieschritt mit einem Insektenelektroantennografieschritt gekoppelt ist und (ii) das Vorhandensein von Schimmelkontamination in Getreide durch Auswertung der Antennenreaktion bei der Retentionszeit, die der eines einzelnen spezifischen Biomarkers entspricht, festgestellt wird, wobei es sich bei dem einzelnen spezifischen Biomarker, der für eine Schimmelkontamination indikativ ist, um 3-Methyl-1-butanol handelt.

2. Verfahren nach Anspruch 1, **dadurch gekennzeichnet, dass** die für die Verwendung in dem Elektroantennografieschritt geeignete Antenne aus der *Plodia interpunctella, Tenbrio molitor* L., *Geotrupes stercorarius* L., *Leptinotarsa decemlineata* (Say), *Drosophila melanogaster* (Meigen), *Musca domestica* L., *Coreus marginatus* L., *Vespula germanica* (Fabricius) und *Apis mellifera* L., vorzugsweise *Drosophila melanogaster, Plodia interpunctella* und *Apis mellifera* umfassenden Gruppe von Insekten ausgewählt ist.

3. Verfahren nach Anspruch 2, **dadurch gekennzeichnet, dass** es sich bei der für die Verwendung im Elektroantennografieschritt geeigneten Antenne um die von *Drosophila melanogaster* handelt.

4. Verfahren nach einem der vorhergehenden Ansprüche, **dadurch gekennzeichnet, dass** Gaschromatografie und Insektenelektroantennografie gleichzeitig durchgeführt werden.

5. Verfahren nach Anspruch 4, **dadurch gekennzeichnet, dass** parallel zur Insektenelektroantennografie eine Flammenionisationsdetektion erfolgt, indem man den Gaschromatografieausstrom in einem Verhältnis von 1:1 zwischen Flammenionisationsdetektor und Elektroantennografiedetektor splittet.

6. Verfahren nach einem der vorhergehenden Ansprüche, **dadurch gekennzeichnet, dass** die Entnahme der gasförmigen Probe aus dem Getreide bei Normaldruck und einer Temperatur von bis zu 40 ± 5°C erfolgt, wobei über mindestens 30 min flüchtige Komponenten von einer Menge von bis zu 25 g Getreide extrahiert werden.

7. Verfahren nach einem der vorhergehenden Ansprüche, **dadurch gekennzeichnet, dass** der Schimmelpilz aus einer *Alternaria alternata, Alternaria brassicola, Aureobasidium pullulans, Aspergillus flavus, Aspergillus niger, Aspergillus ochrachloron, Aspergillus parasiticus, Aspergillus oryzae, Fusarium graminearum, Fusarium culmorum, Fusarium poae, Fusarium sporotrichioides, Penicillium aurantiogriseum, Penicillium chrysogenum, Penicillium citrinum, Penicillium cyclopium, Penicillium digitatum, Penicillium funiculosum, Penicillium roquefortii, Penicillium raistricki, Penicillium viridicatum, Penicillium expansum, Scopulariopsis brevicaulis, Rhizopus oryzae* und *Gusarium* spp. umfassenden Gruppe von Pilzen ausgewählt ist.

8. Verwendung von 3-Methyl-1-butanol als einzelner spezifischer Biomarker für den elektroantennografischen Nachweis einer Schimmelkontamination in gelagertem Getreide, wobei eine elektroantennografische Detektion mit Gaschromatografie gekoppelt ist.

## Revendications

1. Procédé pour la détection de contamination par une moisissure dans du grain stocké, qui comprend la collecte d'un échantillon gazeux du grain et le traitement de l'échantillon collecté par chromatographie en phase gazeuse pour détecter un ou plusieurs composés volatils cibles dans l'échantillon, ***caractérisé par*** (i) le couplage de l'étape de chromatographie en phase gazeuse avec une étape d'électroantennographie d'insecte et (ii) la détermination de la présence de contamination par une moisissure dans le grain par l'évaluation de la réponse antennaire au temps de rétention, correspondant à celui d'un biomarqueur spécifique unique, ledit biomarqueur spécifique unique, indiquant une contamination par une moisissure, étant le 3-méthyl-1-butanol.

2. Procédé selon la revendication 1, ***caractérisé* en ce que** l'antenne appropriée pour une utilisation dans l'étape d'électroantennographie est choisie dans le groupe d'insectes comprenant *Plodia interpunctella, Tenebrio molitor* L., *Geotrupes stercorarius* L., *Leptinotarsa decemlineata* (Say), *Drosophila melanogaster* (Meigen), *Musca domestica* L., *Coreus marginatus* L., *Vespula germanica* (Fabricius), et *Apis mellifera* L., préférablement *Drosophila melanogaster, Plodia interpunctella* et *Apis mellifera.*

3. Procédé selon la revendication 2, ***caractérisé* en ce que** l'antenne appropriée pour une utilisation dans l'étape d'électroantennographie est celle de *Drosophila melanogaster.*

4. Procédé selon l'une quelconque des revendications précédentes, ***caractérisé* en ce *que*** la chromatographie en phase gazeuse et l'électroantennographie d'insecte sont réalisées simultanément.

5. Procédé selon la revendication 4, ***caractérisé en ce qu'***une détection à l'ionisation de flamme est réalisée en parallèle avec l'électroantennographie d'insecte en partageant l'effluent de chromatographie en phase gazeuse entre le détecteur d'ionisation de flamme et le détecteur d'électroantennographie en le rapport 1 : 1.

6. Procédé selon l'une quelconque des revendications précédentes, ***caractérisé* en ce que** la collecte d'un échantillon gazeux du grain est réalisée à pression ambiante et à une température allant jusqu'à 40 ± 5 °C, extrayant des composés volatils d'une quantité de grain allant jusqu'à 25 g pendant au moins 30 min.

7. Procédé selon l'une quelconque des revendications précédentes, ***caractérisé* en ce que** la moisissure est choisie dans un groupe de champignons, comprenant *Alternaria alternata, Alternaria brassicola, Aureobasidium pullulans, Aspergillus flavus, Aspergillus niger, Aspergillus ochrachloron, Aspergillus parasiticus, Aspergillus oryzae, Fusarium graminearum, Fusarium culmorum, Fusarium poae, Fuasarium sporotrichioides, Penicillium aurantiogriseum, Penicillium chrysogenum, Penicillium citrinum, Penicillium cyclopium, Penicillium digitatum, Penicillium funiculosum, Penicillium roquefortii, Penicillium raistricki, Penicillium viridicatum, Penicillium expansum, Scopulariopsis brevicaulis, Rhizopus oryzae* et *Gusarium* spp.

8. Utilisation de 3-méthyl-1-butanol en tant que biomarqueur spécifique unique pour la détection électroantennographique de contamination par une moisissure dans du grain stocké, la détection électroantennographique étant couplée avec une chromatographie en phase gazeuse.
